(19) Europäisches Patentamt
European Patent Office
Office européen des brevets

(11) **EP 4 365 542 B1**

(12) **EUROPEAN PATENT SPECIFICATION**

(45) Date of publication and mention
of the grant of the patent:
**28.01.2026 Bulletin 2026/05**

(21) Application number: **22832225.1**

(22) Date of filing: **01.07.2022**

(51) International Patent Classification (IPC):
*G01B 11/24* (2006.01)   *A61C 9/00* (2006.01)
*A61C 13/00* (2006.01)

(52) Cooperative Patent Classification (CPC):
**A61C 9/006; A61B 5/0088; A61C 9/0053;
A61C 13/0004; G01B 11/25;** G01B 2210/52

(86) International application number:
**PCT/CN2022/103441**

(87) International publication number:
**WO 2023/274413 (05.01.2023 Gazette 2023/01)**

(54) **THREE-DIMENSIONAL SCANNING SYSTEM AND METHOD OF PROCESSING SCANNING DATA**

DREIDIMENSIONALES ABTASTSYSTEM UND VERFAHREN ZUR VERARBEITUNG VON
SCANDATEN

SYSTÈME DE BALAYAGE TRIDIMENSIONNEL ET PROCÉDÉ DE TRAITEMENT DES DONNÉES
DE BALAYAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.07.2021 CN 202110745765**

(43) Date of publication of application:
**08.05.2024 Bulletin 2024/19**

(73) Proprietor: **Shining 3D Tech Co., Ltd.
Hangzhou, Zhejiang 311258 (CN)**

(72) Inventors:
• **MA, Chao**
  **Hangzhou, Zhejiang 311258 (CN)**
• **ZHAO, Xiaobo**
  **Hangzhou, Zhejiang 311258 (CN)**
• **JIANG, Tengfei**
  **Hangzhou, Zhejiang 311258 (CN)**
• **CHEN, Xiaojun**
  **Hangzhou, Zhejiang 311258 (CN)**
• **JIA, Yanming**
  **Hangzhou, Zhejiang 311258 (CN)**

(74) Representative: **Novagraaf Group
Chemin de l'Echo 3
1213 Onex / Geneva (CH)**

(56) References cited:
CN-A- 105 287 039    CN-A- 108 198 235
CN-A- 108 364 257    CN-A- 112 330 732
CN-A- 112 509 018    CN-A- 113 483 695
JP-A- 2020 058 677   JP-B1- 6 208 905
US-A1- 2008 176 188  US-A1- 2013 273 492
US-A1- 2020 405 457  US-A1- 2021 038 350
US-A1- 2021 137 653

EP 4 365 542 B1

## Description

## Technical Field

Embodiments of the present disclosure relate to the technical field of three-dimensional scanning, and in particular, to a three-dimensional scanning system, an auxiliary member, a processing method and apparatus, a device, and a medium.

## Background Art

Internationally, impression three-dimensional scanning is gradually developed to intraoral three-dimensional scanning for the way of acquiring dental model data in the field of dental diagnosis and treatment. An intraoral scanner, also known as an oral digital impression instrument, is mainly used for the intraoral scanning technology. The intraoral scanner is an device which uses a probing-type optical scanning head to directly scan the interior of the oral cavity of a patient so as to obtain information about the three-dimensional shape and color texture of the surfaces of teeth, gums, mucous membranes and other soft and hard tissues in the oral cavity. The intraoral scanner can typically scan cases for restoration, orthodontics and implantation. Whether a case is treated successfully or not is evaluated on the basis of the accuracy of a restored dental crown, the position of an implanted tooth, a brace for orthodontics, and the like.

In order to improve the accuracy of three-dimensional scanning, a more common solution is to use an auxiliary device to assist the intraoral scanner in scanning. For example, an auxiliary device which has a large field of view, such as a scanning bar with recognizable features is implanted. However, the cost of such auxiliary devices is higher than that of the intraoral scanner, and consequently it is more difficult to popularize the auxiliary device in clinics to a wide range. US2021/137653 A1, US2020/405457 A1 and US2021/038350 A1 respectively provide respective corresponding solutions, however, the above mentioned problem remains unsolved.

## Summary of the Invention

(I) Technical Problem To Be Solved.

In order to solve the above technical problems or at least partially solve the above technical problems, embodiments of the present disclosure provide a three-dimensional scanning system, an auxiliary member, a processing method and apparatus, a device, and a medium so as to improve the three-dimensional scanning accuracy and reduce the scanning cost.

(II) Technical Solution

The present invention provides a three-dimensional scanning system as defined in claim 1.

In some embodiments of the present disclosure,

the intraoral scanner is configured to scan the oral cavity to obtain local multi-frame two-dimensional data of the oral cavity;
the scanning data processing device is configured to perform three-dimensional reconstruction on the local multi-frame two-dimensional data of the oral cavity to obtain the local multi-frame three-dimensional data of the oral cavity.

In some embodiments of the present disclosure, the step of determining, by the scanning data processing device, on the basis of the local multi-frame three-dimensional data of the oral cavity and the standard three-dimensional data of the auxiliary member, the overall three-dimensional data of the oral cavity, includes:
performing, by the scanning data processing device, optimized splicing on the local multi-frame three-dimensional data of the oral cavity and the standard three-dimensional data of the auxiliary member to obtain the overall three-dimensional data of the oral cavity.

The present disclosure provides an auxiliary member, the auxiliary member is configured to be mounted on an implant of a dental arch in an oral cavity, and is provided with a mounting portion adapted to the implant;
the auxiliary member is a polyhedron composed of a plurality of irregular polygons, each polygon on the same auxiliary member having a different side length value.

In some embodiments of the present disclosure, the surface of the auxiliary member has no reflective layer.

The present invention also provides a method for processing scanning data as defined in claim 9.

In some embodiments of the present disclosure, the relative motion value obtained from previous splicing includes: a relative motion initial value;
wherein, before constructing, on the basis of the relative motion value of current sampled splicing, the average value of a distance between registration point pairs in the three-dimensional data of current sampled splicing, and a relative motion value obtained from previous splicing, a global optimization energy function, the method further includes:
fully splicing the local multi-frame three-dimensional data of the oral cavity and the standard three-dimensional data of the auxiliary member to determine the relative motion initial value.

In some embodiments of the present disclosure, the iteration stopping condition includes a preset threshold value, and the viewing angle constraint condition includes a preset relative motion value;

wherein, before using the relative motion value obtained from current splicing as the relative motion target value, the method further includes:

determining whether the value of the global optimization energy function is less than or equal to the preset threshold value;

determining whether the relative motion value is less than or equal to the preset motion value; and

in the case where the value of the global optimization energy function is less than or equal to the preset threshold value and the relative motion value is less than or equal to the preset motion value, determining that the global optimization energy function satisfies the iteration stopping co condition and the relative motion value obtained from current splicing satisfies the viewing angle constraint condition.

The present disclosure provides an apparatus for processing scanning data, is configured in a three-dimensional scanning device in a three-dimensional scanning system, the apparatus including:

a data acquisition component, configured to acquire local multi-frame three-dimensional data of an oral cavity and standard three-dimensional data of an auxiliary member; and

an overall three-dimensional data determination component, configured to perform optimized splicing on the local multi-frame three-dimensional data of the oral cavity and the standard three-dimensional data of the auxiliary member to obtain overall three-dimensional data of the oral cavity.

The present disclosure provides a scanning data processing device, including:

a processor; and

a memory, configured to store an executable instruction;

wherein the processor is configured to read the executable instruction from the memory and execute the executable instruction to implement the method for processing scanning data of any of the above.

The present disclosure provides a computer-readable storage medium, the storage medium storing a computer program, the computer program, when executed by a processor, enabling the processor to implement the video display method of any of the above.

(III) Beneficial Effects

The above technical solution provided by embodiments of the present disclosure has the following advantages over the prior art.

After mounting the auxiliary member on an implant of a dental arch in an oral cavity, and obtaining the local multi-frame three-dimensional data of the oral cavity by scanning with the three-dimensional scanning device, the scanning data processing device can optimize, on the basis of standard three-dimensional data of the auxiliary member, the local multi-frame three-dimensional data of the oral cavity. Compared to the way of optimizing, on the basis of framework data obtained by an auxiliary device with a large field of view, three-dimensional scanning data, while the accuracy of scanning data is improved, the optimization cost can be reduced, achieving the purpose of accurately simulating the entire dental arch.

It should be understood that the above general description and the later detailed description are only exemplary and explanatory and do not limit the present disclosure.

**Brief Description of the Drawings**

Accompanying drawings herein are incorporated into a specification and constitute a part of this specification, show embodiments that conform to the present disclosure, and are used for describing a principle of the present disclosure together with this specification.

To describe the technical solutions in the embodiments of the present disclosure or in the prior art more clearly, the following briefly introduces the accompanying drawings for describing the embodiments or the prior art. Apparently, a person of ordinary skill in the art can still derive other drawings from the accompanying drawings without creative efforts.

FIG. 1 is a schematic structural diagram of a three-dimensional scanning system provided by some embodiments of the present disclosure;

FIG. 2 is a schematic structural diagram of an auxiliary member provided by some embodiments of the present disclosure;

FIG. 3 is a schematic structural diagram of an intraoral scanner provided by some embodiments of the present disclosure;

FIG. 4 is a flowchart of a method for processing scanning data provided by some embodiments of the present disclosure;

FIG. 5 is a schematic structural diagram of an apparatus for processing scanning data provided by some embodiments of the present disclosure; and

FIG. 6 is a schematic structural diagram of a scanning data processing device provided by some embodiments of the present disclosure.

**Detailed Description of the Invention**

To make the objectives, technical solutions, and advantages of embodiments of the present disclosure clearer, the following clearly and completely describes the technical solutions in the embodiments of the present disclosure. Apparently, the described embodiments are some rather than all of the embodiments of the present disclosure. All other embodiments obtained by a person of ordinary skill in the art based on the embodiments of the present disclosure without creative efforts shall fall within the protection scope of the present disclosure.

In the related art, when an intraoral scanner is used for collecting images of the interior of an oral cavity, a three-dimensional scanning technology is adopted for a probing-type optical scanning head of the intraoral scanner to probe the oral cavity to directly scan the oral cavity of a patient so as to obtain information about the three-dimensional shape and color texture of the surfaces of teeth, gums, mucous membranes and other soft and hard tissues in the oral cavity. Due to the confined environment inside the oral cavity, the size of the probing-type optical scanning head is limited. In general, the size of the probing-type scanning head is controlled within 30 mm, so that the probing-type optical scanning head can only acquire images of up to two teeth at a time. However, in the dental industry, in most cases, it needs to acquire data of whole teeth and gums in the oral cavity in the process of designing and making teeth, and then it needs to splice the images of teeth acquired at a time to form three-dimensional spatial data consistent with the whole teeth in a mouth.

The intraoral scanner can typically scan cases for restoration, orthodontics and implantation. Whether a case is treated successfully or not is evaluated on the basis of the accuracy of a restored dental crown, the position of an implanted tooth, a brace for orthodontics, and the like.

In order to improve the accuracy of a restored dental crown, the position of an implanted tooth, and a brace for orthodontics, regular objects such as circular truncated cones and square blocks are used as standard members to assist in optimizing the three-dimensional scanning data obtained by scanning with the intraoral scanner. However, the regular objects such as circular truncated cones and square blocks often lead to splicing errors, and the volume of the circular truncated cones and square blocks cannot completely simulate and express the accuracy of data of full-jaw teeth of the whole dental arch. That is, the above standard members can be used for evaluating the accuracy of cases for restoring a single tooth or a few teeth, and cannot be used for evaluating the accuracy of orthodontic cases of the full-jaw teeth.

Further, in order to improve the accuracy of the orthodontic cases of the full-jaw teeth, an auxiliary device is used in the related art to perform framework scanning on the full dental arch and the like, and after obtaining the framework accuracy of the full dental arch, the intraoral scanner is then used to perform fine complementary scanning on the full dental arch. This approach ensures the scanning accuracy of the full dental arch while pursuing the details of data. For example, mark points are pasted in the oral cavity, a scanning bar with mark points or a scanning bar with recognizable features is implanted in the oral cavity, and an auxiliary device with a large field of view is used to acquire the position of each mark point or feature with high accuracy as framework data. Then, the intraoral scanner is used to scan the teeth, the gums, and the various mark points and features, and the framework data is used as reference data to perform optimized splicing on the three-dimensional scanning data obtained by the intraoral scanner, so as to obtain target scanning data with high accuracy of the full dental arch, thereby improving the accuracy of the orthodontic case of the full-jaw teeth. However, in practical application, the cost of the above auxiliary device is higher than that of the intraoral scanner, although the above auxiliary device meets the accuracy requirements of the orthodontic cases of the full-jaw teeth, it is more difficult to popularize the auxiliary device to clinics to a wide range, failing to benefit general patients.

In summary, the way of using the regular objects such as the circular truncated cones and the square blocks as standard members to assist in optimizing the three-dimensional scanning data obtained by scanning with the intraoral scanner is not able to evaluate the accuracy of the orthodontic cases of the full-jaw teeth. In view of the way of using the auxiliary device with a large field of view to assist in optimizing the three-dimensional scanning data obtained by scanning with the intraoral scanner, the cost of the auxiliary device is high, and consequently it is not possible to popularize and use the auxiliary device.

In order to solve the above problems, embodiments of the present disclosure provide a three-dimensional scanning system, an auxiliary member, a processing method and apparatus, a device, and a medium. Compared to the way of optimizing, on the basis of the framework data obtained by the auxiliary device with a large field of view, three-dimensional scanning data, optimizing, on the basis of the standard three-dimensional data of the auxiliary member, local multi-frame three-dimensional data of the oral cavity has the advantages that while the accuracy of scanning data is improved, the optimization cost can be reduced, achieving the purpose of accurately simulating the entire dental arch, meeting the accuracy requirements for restoring a dental crown, performing orthodontic treatment on a tooth position and a brace for orthodontics, and facilitating popularization and application.

A three-dimensional scanning system provided by an embodiment of the present disclosure is first described below.

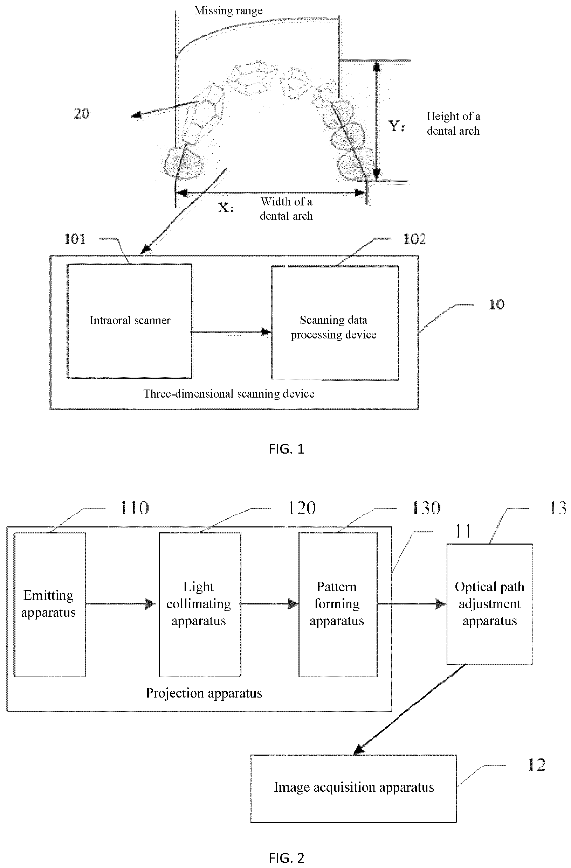

FIG. 1 is a schematic structural diagram of a three-dimensional scanning system provided by some embodiments of the present disclosure. The three-dimensional scanning system includes a three-dimensional scanning device 10 and auxiliary members 20.

The auxiliary member 20 is mounted on an implant of a dental arch in an oral cavity.

The three-dimensional scanning device 10 performs three-dimensional scanning on the oral cavity to obtain local multi-frame three-dimensional data of the oral cavity, and determines, on the basis of the local multi-frame three-dimensional data of the oral cavity and standard three-dimensional data of the auxiliary member, overall three-dimensional data of the oral cavity.

In some embodiments of the present disclosure, the auxiliary member refers to a polyhedron mounted on the implant of the dental arch, and the auxiliary member may be designed in advance by a scanning data processing device on the basis of structural characteristics of a diseased tooth in the oral cavity and structural characteristics of teeth surrounding the diseased tooth. The structural characteristics of a tooth may include: characteristic data such as the morphology and volume of the tooth. In some embodiments of the present disclosure, the auxiliary member may be, but is not limited to, structures such as quadrangular prisms, hexagonal prisms, and the like.

In order to improve the splicing smoothness between the auxiliary members, symmetry and repetition between polygonal planes on the auxiliary members need to be reduced. In some embodiments of the present disclosure, each plane of the auxiliary member may be designed as an irregular polygon by means of computer aided design (CAD) software on the basis of structural characteristics of the dental arch, and the side length value of each polygon on the same auxiliary member is different. In this way, the shape of each polygon on the auxiliary member is different, and the side length value of each polygon is also different. By designing the auxiliary member as an irregular polygon, the auxiliary member can adapt to the structural characteristics of the dental arch, thereby improving the splicing smoothness between the auxiliary members, making the spliced auxiliary members fit well the full dental arch, facilitating processing and improving the characteristic of making detection easy.

In some embodiments of the present disclosure, before mounting the auxiliary member on the implant of the dental arch in the oral cavity, the position of the implant of the dental arch can be predetermined on the basis of the lesion of teeth in the oral cavity, and an appropriately sized auxiliary member is selected on the basis of the size of the diseased tooth and the distance between the diseased tooth and teeth surrounding the diseased tooth. Further, on the basis of threads around the implant and threads of the auxiliary member, the auxiliary member is mounted on the implant at the corresponding position shown in FIG. 1 to complete the operation of mounting the auxiliary member in the oral cavity.

The three-dimensional scanning device 10 includes an intraoral scanner 101 and a scanning data processing device 102.

The intraoral scanner 101 is configured to scan the oral cavity to obtain local multi-frame two-dimensional data of the oral cavity.

The scanning data processing device 102 is configured to perform three-dimensional reconstruction on the local multi-frame two-dimensional data of the oral cavity to obtain the local multi-frame three-dimensional data of the oral cavity.

The scanning data processing device 102 is further configured to determine, on the basis of the local multi-frame three-dimensional data of the oral cavity and the standard three-dimensional data of the auxiliary member, the overall three-dimensional data of the oral cavity.

In order to improve the optimization accuracy of the overall dimensional data of the oral cavity, in some embodiments of the present disclosure, the step of determining, by the scanning data processing device, on the basis of the local multi-frame three-dimensional data of the oral cavity and the standard three-dimensional data of the auxiliary member, the overall three-dimensional data of the oral cavity may include:
performing, by the scanning data processing device, optimized splicing on the local multi-frame three-dimensional data of the oral cavity and the standard three-dimensional data of the auxiliary member to obtain the overall three-dimensional data of the oral cavity.

In some embodiments of the present disclosure, after the auxiliary member is mounted on the implant of the dental arch in the oral cavity, a probing-type optical scanning head of the intraoral scanner may probe the oral cavity to scan the oral cavity to obtain the local multi-frame two-dimensional data of the oral cavity. The local multi-frame two-dimensional data refers to scanned point cloud data, which may include the local multi-frame two-dimensional data of teeth in the oral cavity. Further, the intraoral scanner sends the local multi-frame two-dimensional data of the oral cavity to the scanning data processing device, and the scanning data processing device performs three-dimensional reconstruction on the local multi-frame two-dimensional data to obtain the local multi-frame three-dimensional data of the oral cavity corresponding to the local multi-frame two-dimensional data of the oral cavity, in order to further perform optimized splicing on the local multi-frame three-dimensional data of the oral cavity and the standard three-dimensional data of the auxiliary member to obtain the overall three-dimensional data of the oral cavity.

In some embodiments of the present disclosure, the standard three-dimensional data of the auxiliary member refers to feature point data of each polygon on the auxiliary member determined by the scanning data processing device when designing the auxiliary member. When the scanning data processing device determines the overall three-dimensional data, the standard three-dimensional data of the auxiliary member may be simulated into a point cloud space and the overall three-dimensional data of the oral cavity is determined in combination of the local multi-frame three-dimensional data of the oral cavity.

In some embodiments of the present disclosure, the scanning data processing device may splice the local multi-frame three-dimensional data of the oral cavity and optimize the spliced local multi-frame three-dimensional data of the oral cavity on the basis of the standard three-dimensional data of the auxiliary member to obtain the overall three-dimensional data of the oral cavity.

In some embodiments of the present disclosure, the scanning data processing device may perform registration on the local multi-frame three-dimensional data of the oral cavity, as well as perform registration on the local multi-frame three-dimensional data of the oral cavity and the standard three-dimensional data of the auxiliary member, to obtain registration point pairs composed of the local three-dimensional data and registration point pairs composed of the standard three-dimensional data of the auxiliary member and the local three-dimensional data. Further, the registration point pair of the local multi-frame three-dimensional data of the oral cavity and the registration point pair of the standard three-dimensional data of the auxiliary member are spliced to obtain the spliced local multi-frame three-dimensional data of the whole oral cavity, and the spliced local multi-frame three-dimensional data includes three-dimensional data of the dental arch and the standard three-dimensional data of the auxiliary member. Optimizing, on the basis of the standard three-dimensional data of the auxiliary member, the spliced local multi-frame three-dimensional data can determine the overall three-dimensional data of the oral cavity, i.e., obtaining the optimized scanning data of the oral cavity.

FIG. 2 is a schematic structural diagram of an embodiment of an intraoral scanner. The intraoral scanner 101 includes a projection apparatus 11, an image acquisition apparatus 12 and an optical path adjustment apparatus 13.

The projection apparatus 11, as an embodiment, includes an emitting apparatus 110, a light collimating apparatus 120 and a pattern forming apparatus 130.

The emitting apparatus 110 is configured to emit preset light rays, and the preset light rays include at least two monochromatic light rays.

The light collimating apparatus 120 is configured to homogenize the preset light rays.

The pattern forming apparatus 130 is configured to project the homogenized preset light rays in a structured light pattern.

The optical path adjustment apparatus 13 is configured to change the transmission path of the structured light pattern to project the structured light pattern to the dental arch and project the structured light pattern modulated by the dental arch to the image acquisition apparatus.

The image acquisition apparatus 12 is configured to perform beam splitting on the structured light pattern modulated by the dental arch, and acquire, by different cameras, a plurality of structured light patterns after beam splitting to obtain two-dimensional scanning data of the dental arch.

The emitting apparatus 110 may employ an emitting apparatus composed a laser emitting apparatus, a cylindrical lens and a color combining apparatus. In some embodiments of the present disclosure, the light collimating apparatus 120 may include at least one fly-eye lens. The fly-eye lens homogenizes the preset light rays to project the homogenized preset light rays to the pattern forming apparatus.

In some embodiments of the present disclosure, the pattern forming apparatus 130 may be a color grating filter. The color grating filter is configured to transmit the homogenized preset light rays to generate a structured light pattern projected in a form of stripes.

In some embodiments of the present disclosure, the optical path adjustment apparatus 13 may include a reflecting apparatus to change the transmission path of the structured light pattern so as to project the structured light pattern to the dental arch, and to project the structured light pattern modulated by the dental arch to the image acquisition apparatus. The reflecting apparatus may be a reflector or other apparatuses with specific reflective functions.

In some embodiments of the present disclosure, the image acquisition apparatus 12 may include a color separating prism and two cameras. The color separating prism performs beam splitting on the structured light pattern, collects, by one of the cameras, one way of light rays after beam splitting, collects, by the other camera, one way of light rays after beam splitting, and uses the two ways of collected light rays as the two-dimensional scanning data of the dental arch. Therefore, in some embodiments of the present disclosure, when scanning the interior of the oral cavity using the above intraoral scanner, the preset light rays are homogenized by means of the light collimating apparatus so that the preset light rays can be homogenized at an energy level to achieve a higher light energy utilization rate and uniform illumination over a larger area, which is conducive to projecting the uniform preset light rays, avoiding the phenomenon of diffraction spots, and improving the utilization rate of the light rays. Moreover, the plurality of structured light patterns after beam splitting are acquired by different cameras so as to distinguish the structured light patterns after beam splitting, avoiding mutual interference between different structured light patterns, and improving the accuracy of the scanning data.

Certainly, the projection apparatus 11 may also be referred to as Digital Light Processing (DLP) or Liquid Crystal on Silicon (LCOS).

According to the technical solution of the embodiment of the present disclosure, after mounting the auxiliary member on an implant of a dental arch in an oral cavity, and obtaining local multi-frame three-dimensional data of the oral cavity by scanning with the three-dimensional scanning device, the scanning data processing device can optimize, on the basis of the standard three-dimensional data of the auxiliary member, the local multi-frame three-dimensional data of the oral cavity. Compared to the way of optimizing, on the basis of framework data obtained by the auxiliary device with a large field of view, three-dimensional scanning data, while the accuracy of scanning data is improved, the optimization cost can be reduced, achieving the purpose of accurately simulating the entire dental arch.

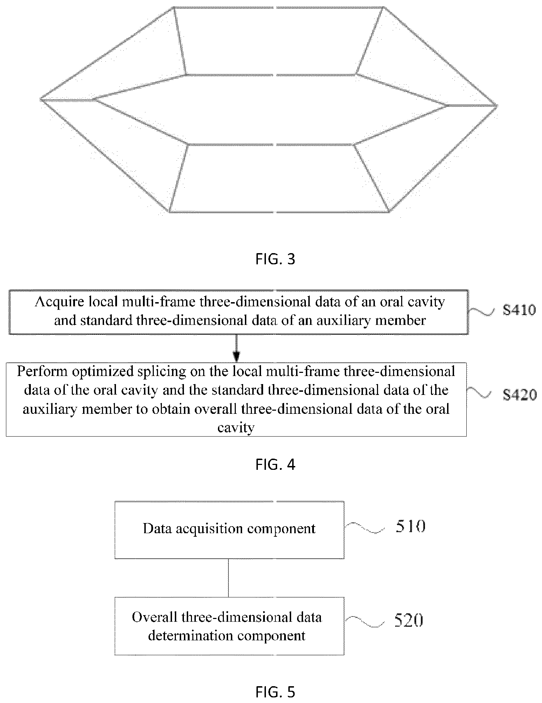

FIG. 3 is a schematic structural diagram of an auxiliary member provided by some embodiments of the present disclosure. With reference to FIG. 3, the auxiliary member is configured to be mounted on an implant of a dental arch in an oral cavity, and is provided with a mounting portion adapted to the implant. The auxiliary member is a polyhedron composed of a plurality of irregular polygons, and each polygon on the same auxiliary member has a different side length value.

In some embodiments of the present disclosure, the structural characteristics of the auxiliary member are adapted to the structural characteristics of the full dental arch. The auxiliary member may be a hexagonal prism as shown in FIG. 3. A single auxiliary member can fit well on the full dental arch, and the presence of fewer polygonal planes of symmetry and repetition on the auxiliary member improves the splicing smoothness between the auxiliary members, so that the spliced auxiliary members can fit well on the full dental arch. For the auxiliary member fixed at different positions on the full dental arch, changing the side length size of the auxiliary member changes the structural characteristics of the auxiliary member so as to reduce the same features on the multiple planes on the auxiliary member.

In some embodiments of the present disclosure, each plane of the auxiliary member may be designed as an irregular polygon by means of computer aided design (CAD) software on the basis of structural characteristics of the dental arch, and the side length value of each polygon on the same auxiliary member is different. In this way, each polygon on the auxiliary member has a different shape and each polygon has a different side length value, which can improve the splicing smoothness between the auxiliary members, and symmetry and repetition between polygonal planes on the auxiliary member need to be reduced.

In some embodiments of the present disclosure, the surface of the auxiliary member has no reflective layer. For example, the surface of the auxiliary member is free of sandblasting, etc. Since the surface of the auxiliary member has no reflective layer, the surface reflection of the auxiliary member can be reduced, and the scanning efficiency and scanning accuracy can be further improved.

According to the technical solution provided by the embodiment of the present disclosure, the auxiliary member is a polyhedron composed of a plurality of irregular polygons. Compared to a standard member in the shape of a circular truncated cone and the shape of a square block, the plurality of auxiliary members can be well spliced on the basis of the structural characteristics of the dental arch, no splicing error occurs, and it is easy to make the auxiliary member. Moreover, the surface of the auxiliary member has no reflective layer, which can reduce the surface reflection of the auxiliary member, and can further improve the scanning efficiency and scanning accuracy, and the cost is low, which is conducive to popularization and application.

FIG. 4 is a flowchart of a method for processing scanning data provided by some embodiments of the present disclosure.

As shown in FIG. 4, the method for processing scanning data is applied to the scanning data processing device described above, and the method may specifically include the following steps.

S410: Acquire local multi-frame three-dimensional data of an oral cavity and standard three-dimensional data of an auxiliary member.

In some embodiments of the present disclosure, the local multi-frame three-dimensional data of the oral cavity may be obtained by reconstructing, by the scanning data processing device in a three-dimensional scanning device, local multi-frame two-dimensional data of the oral cavity obtained by scanning with an intraoral scanner. The local multi-frame three-dimensional data of the oral cavity may include local multi-frame three-dimensional data of teeth in the oral cavity and local multi-frame three-dimensional data of the auxiliary member. The standard three-dimensional data of the auxiliary member may be feature point data of each polygon on the auxiliary member determined by the scanning data processing device when designing the auxiliary member.

In some embodiments of the present disclosure, the auxiliary member is mounted on an implant of a dental arch in the oral cavity, the auxiliary member is a polyhedron composed of a plurality of irregular polygons, each polygon on the same auxiliary member has a different side length value, and each polygon on different auxiliary members mounted on different implants has a different side length value.

S420: Perform optimized splicing on the local multi-frame three-dimensional data of the oral cavity and the standard three-dimensional data of the auxiliary member to obtain overall three-dimensional data of the oral cavity.

As described previously, the overall three-dimensional data of the oral cavity refers to optimized scanning data of the oral cavity.

In some embodiments of the present disclosure, performing optimized splicing on the local multi-frame three-dimensional data of the oral cavity and the standard three-dimensional data of the auxiliary member to obtain overall three-dimensional data of the oral cavity may include:

step 1: performing sampled splicing on the local multi-frame three-dimensional data of the oral cavity and the standard three-dimensional data of the auxiliary member to obtain a relative motion value of current sampled splicing and an average value of a distance between registration point pairs in the three-dimensional data of current sampled splicing;

step 2: constructing, on the basis of the relative motion value of current sampled splicing, the average value of a distance between registration point pairs in the three-dimensional data of current sampled splicing, and a relative motion value obtained from previous splicing, a global optimization energy function;

step 3: in the case where the global optimization energy function satisfies an iteration stopping condition and the relative motion value obtained from current splicing satisfies a viewing angle constraint condition, using the relative motion value obtained from current splicing as a relative motion target value; and

step 4: splicing, on the basis of the relative motion target value, the local multi-frame three-dimensional data of the oral cavity to determine the overall three-dimensional data of the oral cavity.

For step 1, in some embodiments of the present disclosure, the scanning data processing device in the three-dimensional scanning device may select arbitrary sampled point pairs of the local multi-frame three-dimensional data of the oral cavity and the standard three-dimensional data of the auxiliary member, and perform sampled splicing on the sampled point pairs to obtain a relative motion value of current sampled splicing and an average value of a distance between registration point pairs in the three-dimensional data of current sampled splicing.

In some embodiments of the present disclosure, the relative motion value may refer to a relative pose between the registration point pairs composed of the local three-dimensional data, and a relative pose between the registration point pairs composed of the local three-dimensional data and the standard three-dimensional data of the auxiliary member.

In some embodiments of the present disclosure, the average value of a distance may include a distance between the registration point pairs composed of the local three-dimensional data and a distance between the registration point pairs composed of the local three-dimensional data and the standard three-dimensional data of the auxiliary member.

In some embodiments, step 1 may specifically include:

step 11: performing sampled splicing on the local three-dimensional data to obtain a first relative motion value, performing sampled splicing on the local three-dimensional data and the standard three-dimensional data of the auxiliary member to obtain a second relative motion value, and forming a relative motion value of current sampled splicing by the first relative motion value and the second relative motion value; and

step 12: calculating a first distance value between registration point pairs in the local multi-frame three-dimensional data of sampled splicing, calculating a second distance value between registration point pairs of the local multi-frame three-dimensional data of sampled splicing and the standard three-dimensional data of the auxiliary member, and forming an average value of a distance by the first distance value and the second distance value.

In some embodiments, on the basis of a spatial relationship of the local three-dimensional data of sampled splicing, a rotational translation matrix of the sampled local three-dimensional data may be calculated to obtain the first relative motion value, a rotational translation matrix of the local three-dimensional data of sampled splicing and the sampled standard three-dimensional data of the auxiliary member may be calculated to obtain the second relative motion value, and the relative motion value is formed by the first relative motion value and the second relative motion value.

In some embodiments of the present disclosure, the rotational translation matrix may include a rotation matrix (Rxn, Ryn, and Rzn) and a translation matrix (xm, ym, and zm). Rxn, Ryn, and Rzn are rotation axes in x, y, and z directions, respectively. xm, ym, and zm are vectors in x, y, and z directions, respectively.

In some embodiments, registration may be performed on the basis of a rigid registration algorithm in an iterative manner, a registration algorithm using an annealing and soft correspondence manner, a similarity measurement method, and other methods to obtain the above registration point pairs. Further, a Euclidean distance between the registration point pairs in the local multi-frame three-dimensional data of sampled splicing is calculated, and used as a first distance, a Euclidean distance between registration point pairs of the local multi-frame three-dimensional data of sampled splicing and the standard three-dimensional data of the auxiliary member is calculated and used as a second distance, and an average value of a distance is formed by an average sum of the first distance value and the second distance value.

In some embodiments of the present disclosure, in the process of performing sampled splicing on the local three-dimensional data, and performing sampled splicing on the local three-dimensional data and the standard three-dimensional data of the auxiliary member, a visual constraint condition expression equation may be generated on the basis of a rotation angle and a rotation axis of the local multi-frame three-dimensional data of the oral cavity and the standard three-dimensional data of the auxiliary member when transformed from a previous angle of view to the current angle of view; on the basis of a low-rank sparse matrix constructed according to the relative motion initial value of the registration point pair at any two angles of view, as well as a translation vector of the registration point pair in the rotational translation matrix at any two angles of view and the Cauchy weight function, a weight matrix is constructed; and on the basis of the visual constraint condition expression equation, the low-rank sparse matrix, and the weight matrix, a mathematical model of the global motion optimization problem is constructed. Further, the mathematical model of the global motion optimization problem is processed to obtain an expression of the optimization problem, and the expression of the optimization problem is solved to obtain the relative motion value of current sampled splicing. In some embodiments of the present disclosure, the mathematical model of the global motion optimization problem may be processed with convex relaxation to obtain an optimization problem expression, and the optimization problem expression may be solved using a Lagrange multiplier method to obtain the relative motion value of current sampled splicing.

For step 2, in some embodiments of the present disclosure, after the scanning data processing device in the three-dimensional scanning device acquires the average value of a distance and the relative motion value of current sampled splicing, on the basis of the relative motion value obtained from current splicing, the average value of a distance between registration point pairs in the three-dimensional data of current sampled splicing, and a relative motion value obtained from previous splicing, a global optimization energy function may be constructed. The relative motion value obtained from previous splicing may be the relative motion value obtained from last splicing in which sampled splicing is completed.

In some embodiments of the present disclosure, the relative motion value obtained from previous splicing may be the relative motion value obtained from first splicing, and the relative motion value obtained from first splicing is used as the relative motion initial value.

In some embodiments, the relative motion value obtained from previous splicing includes: a relative motion initial value.

Correspondingly, before step 2, the method further includes:
fully splicing the local multi-frame three-dimensional data of the oral cavity and the standard three-dimensional data of the auxiliary member to determine the relative motion initial value.

Correspondingly, step 2 may include:
constructing, on the basis of the relative motion value obtained from current splicing, the average value of a distance between registration point pairs in the three-dimensional data of current sampled splicing, and the relative motion initial value, a global optimization energy function.

In some embodiments of the present disclosure, the relative motion initial value may refer to an initial relative pose between the registration point pairs composed of the local three-dimensional data, and an initial relative pose between the registration point pairs composed of the local three-dimensional data and the standard three-dimensional data of the auxiliary member.

In some embodiments, the scanning data processing device in the three-dimensional scanning device may fully splice the local three-dimensional data corresponding to the local two-dimensional data obtained by scanning to obtain a first relative motion initial value, and fully splice the local three-dimensional data and the standard three-dimensional data of the auxiliary member to obtain a second relative motion initial value, and a relative motion initial value is formed by the first relative motion value and the second relative motion value.

In other embodiments of the present disclosure, the relative motion value obtained from previous splicing may be formed by the first relative motion value and the second relative motion value obtained from previous sampled splicing. That is, on the basis of the relative motion value obtained from previous splicing, sampled splicing is performed on the local three-dimensional data under the times of previous splicing to obtain a first relative motion value. Sampled splicing is performed on the local three-dimensional data and the standard three-dimensional data of the auxiliary member to obtain a second relative motion value. A relative motion value of current sampled splicing is formed by the first relative motion value under the times of previous splicing and the second relative motion value.

On the basis of the above description, the expression of the global optimization energy function is:

$$E = \nabla E(i,j) + \sum D(u,v)$$

E refers to the global optimization energy function, $\nabla E(ij)$ refers to a difference between the relative motion value of the registration point pair obtained from current splicing and the relative motion value of the registration point pair obtained from previous splicing, i and j are two angles of view, respectively, $\sum D(u,v)$ refers to an average value of a distance obtained from current splicing, and u and v are sequence position expressions of all registration point pairs of the dental arch, respectively.

For step 3, in some embodiments of the present disclosure, after the scanning data processing device in the three-dimensional scanning device constructs the global optimization energy function, it can be determined whether the global optimization energy function satisfies an iteration stopping condition and whether the relative motion value obtained from current splicing satisfies a viewing angle constraint condition. If the global optimization energy function satisfies the iteration stopping condition and the relative motion value obtained from current splicing satisfies the viewing angle constraint condition, the relative motion value obtained from current splicing is used as a relative motion target value.

In some embodiments, the iteration stopping condition includes a preset threshold value, and the viewing angle constraint condition includes a preset relative motion value.

Correspondingly, before step 3, the method further includes:

> step 31: determining whether the value of the global optimization energy function is less than or equal to the preset threshold value;
> step 32: determining whether the relative motion value satisfies a preset relative motion relationship; and
> step 33: in the case where the value of the global optimization energy function is less than or equal to the preset threshold value and the relative motion value satisfies the preset relative motion relationship, determining that the global optimization energy function satisfies the iteration stopping condition and the relative motion value obtained from current splicing satisfies the viewing angle constraint condition.

The preset threshold value may be a preset value for determining whether the global optimization energy function satisfies the iteration stopping condition. In some embodiments of the present disclosure, the preset threshold value may be a value such as 0.01, 0.02, etc., and is not limited herein.

The preset relative motion relationship may be a relationship used to determine whether the relative motion value obtained from current splicing is stable. In some embodiments of the present disclosure, the preset relative motion relationship may characterize that a rotational translation matrix between adjacent frames of local three-dimensional data is less than or equal to the preset value, and that a rotational translation matrix between the currently spliced local multi-frame three-dimensional data and the standard three-dimensional data of the auxiliary member is less than or equal to the preset value. In some embodiments of the present disclosure, the preset relative motion relationship may be determined on the basis of the rotation angle and the rotation axis during transforming from a viewing angle of previous splicing to a viewing angle of current splicing.

In some embodiments of the present disclosure, after the scanning data processing device in the three-dimensional scanning device constructs the global optimization energy function, the value of the global optimization energy function is compared with the preset threshold value and the relative motion value is compared with the preset relative motion relationship, if the value of the global optimization energy function is less than or equal to the preset threshold value and the relative motion value satisfies the preset relative motion relationship, it is determined that the global optimization energy function satisfies the iteration stopping condition and the relative motion value obtained from current splicing satisfies the viewing angle constraint condition. Further, the scanning data processing device takes the relative motion value obtained from current splicing as the relative motion target value.

In some embodiments of the present disclosure, if the value of the global optimization energy function is greater than the preset threshold value, the global optimization energy function does not satisfy the iteration stopping condition, or, if the relative motion value does not satisfy the preset relative motion relationship, the relative motion value obtained from current splicing does not satisfy the viewing angle constraint condition, the iteration of the global optimization energy function continues to update the relative motion initial value in the global optimization energy function and the weight matrix in the visual constraint condition expression equation is updated and iterated to update the relative motion value and the average value of a distance until the global optimization energy function satisfies the iteration stopping condition and the relative motion value obtained from current splicing satisfies the viewing angle constraint condition, and the relative motion target value is obtained.

For step 4, in some embodiments of the present disclosure, after the scanning data processing device in the three-dimensional scanning device obtains the relative motion target value, the global motion target value of point cloud of each viewing angle can be determined according to the relative motion target value, and all the local multi-frame three-dimensional data is spliced according to the global motion target value to obtain the overall three-dimensional data of the oral cavity.

As a result, by means of steps 1-4, the global optimization energy function can be constructed, and on the basis of the global optimization energy function, the overall three-dimensional data of the oral cavity can be accurately calculated.

According to the technical solution provided by the embodiment of the present disclosure, the three-dimensional scanning device can acquire the local multi-frame three-dimensional data of the oral cavity and the standard three-dimensional data of the auxiliary member, and perform optimized splicing on the local multi-frame three-dimensional data of the oral cavity and the standard three-dimensional data of the auxiliary member to obtain the overall three-dimensional data of the oral cavity, so that the overall three-dimensional data of the oral cavity is determined. Due to the above method, the overall three-dimensional data of the oral cavity can be calculated accurately and efficiently, and the overall three-dimensional data of the oral cavity is three-dimensional data with higher accuracy, which achieves the purpose of accurately simulating the entire dental arch, meets the accuracy requirements of restoring a dental crown, performing orthodontic treatment on a tooth position, and a brace for orthodontics, and facilitates popularization and application.

FIG. 5 is a schematic structural diagram of an apparatus for processing scanning data provided by some embodiments of the present disclosure, and the apparatus is configured in a scanning data processing device. With reference to FIG. 5, the apparatus includes:

a data acquisition component 510, configured to acquire local multi-frame three-dimensional data of an oral cavity and standard three-dimensional data of an auxiliary member; and

an overall three-dimensional data determination component 520, configured to perform optimized splicing on the local multi-frame three-dimensional data of the oral cavity and the standard three-dimensional data of the auxiliary member to obtain overall three-dimensional data of the oral cavity.

According to the technical solution provided by the embodiment of the present disclosure, a three-dimensional scanning device can acquire the local multi-frame three-dimensional data of the oral cavity and the standard three-dimensional data of the auxiliary member, and perform optimized splicing on the local multi-frame three-dimensional data of the oral cavity and the standard three-dimensional data of the auxiliary member to obtain the overall three-dimensional data of the oral cavity, so that the overall three-dimensional data of the oral cavity is determined. Due to the above method, the overall three-dimensional data of the oral cavity can be calculated accurately and efficiently, and the overall three-dimensional data of the oral cavity is three-dimensional data with higher accuracy, which achieves the purpose of accurately simulating the entire dental arch, meets the accuracy requirements of restoring a dental crown, and performing orthodontic treatment on a tooth position, and a brace for orthodontics, and facilitates popularization and application.

In some embodiments of the present disclosure, the overall three-dimensional data determination component 520 is configured to: perform sampled splicing on the local multi-frame three-dimensional data of the oral cavity and the standard three-dimensional data of the auxiliary member to obtain a relative motion value of current sampled splicing and an average value of a distance between registration point pairs in the three-dimensional data of current sampled splicing;

construct, on the basis of the relative motion value of current sampled splicing, the average value of a distance between registration point pairs in the three-dimensional data of current sampled splicing, and a relative motion value obtained from previous splicing, a global optimization energy function;
in the case where the global optimization energy function satisfies an iteration stopping condition and the relative motion value obtained from current splicing satisfies a viewing angle constraint condition, use the relative motion value obtained from current splicing as a relative motion target value; and
splice, on the basis of the relative motion target value, the local multi-frame three-dimensional data of the oral cavity to determine the overall three-dimensional data of the oral cavity.

In some embodiments of the present disclosure, the relative motion value obtained from previous splicing includes: a relative motion initial value.
Correspondingly, the overall three-dimensional data determination component 520 is further configured to fully splice the local multi-frame three-dimensional data of the oral cavity and the standard three-dimensional data of the auxiliary member to determine the relative motion initial value.
In some embodiments of the present disclosure, the iteration stopping condition includes a preset threshold value, and the viewing angle constraint condition includes a preset relative motion value.
Correspondingly, the overall three-dimensional data determination component 520 is further configured to: determine whether the value of the global optimization energy function is less than or equal to the preset threshold value;

determine whether the relative motion value satisfies a preset relative motion relationship; and
in the case where the value of the global optimization energy function is less than or equal to the preset threshold value and the relative motion value satisfies the preset relative motion relationship, determine that the global optimization energy function satisfies the iteration stopping condition and the relative motion value obtained from current splicing satisfies the viewing angle constraint condition.

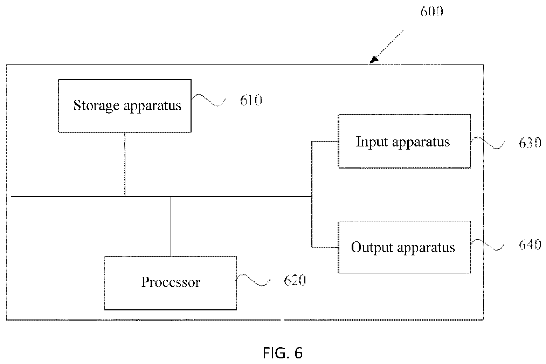

With reference to FIG. 6, the present embodiment provides a scanning data processing device 600, including: one or more processors 620; a storage apparatus 610, configured to store one or more programs, the one or more programs, when executed by the one or more processors 620, enabling the one or more processors 620 to implement the method for processing scanning data provided by the embodiment of the present disclosure, including:

acquiring local multi-frame three-dimensional data of an oral cavity and standard three-dimensional data of an auxiliary member; and
performing optimized splicing on the local multi-frame three-dimensional data of the oral cavity and the standard three-dimensional data of the auxiliary member to obtain overall three-dimensional data of the oral cavity.

Certainly, a person skilled in the art may understand that the processor 620 may also realize the technical solution of the method for processing scanning data provided in any embodiment of the present disclosure.
The scanning data processing device 600 shown in FIG. 6 is merely an example, and should not impose any limitation on the functions and scope of use of the embodiments of the present disclosure.
As shown in FIG. 6, the scanning data processing device 600 includes a processor 620, a storage apparatus 610, an input apparatus 630, and an output apparatus 640. The number of the processor 620 in the device may be one or more, and one processor 620 is taken as an example in FIG. 6. The processor 620, the storage apparatus 610, the input apparatus 630, and the output apparatus 640 in the device may be connected via a bus or in other ways, and connection via a bus 650 is taken as an example in FIG. 6.

The storage apparatus 610, as a computer-readable storage medium, may be configured to store software programs, computer-executable programs, and components, such as program instructions/components corresponding to the method for processing scanning data in the embodiment of the present disclosure (e.g., the data acquisition component and the overall three-dimensional data determination component in the apparatus for processing scanning data ).

The storage apparatus 610 may primarily include a program storage area and a data storage area. The program storage area may store an operating system, and an application program required for at least one function. The data storage area may store data created on the basis of use of a terminal, and the like. In addition, the storage apparatus 610 may include a high-speed random access memory, and may also include a non-volatile memory, such as at least one disk memory device, a flash device, or other non-volatile solid state memory devices. In some examples, the storage apparatus 610 may further include memories that are remotely arranged relative to the processor 620, and these remote memories may be connected to the device via a network. Examples of the above networks include, but are not limited to, the Internet, an enterprise intranet, a local area network, a mobile communications network, and combinations thereof.

The input apparatus 630 may be configured to receive input number or character information, and to generate inputs of key signals related to user settings of the device as well as function control, for example, at least one of a mouse, a keyboard, and a touch screen may be included. The output apparatus 640 may include a display device such as a display screen.

The present embodiment provides a storage medium comprising computer-executable instructions, the computer-executable instructions, when executed by a computer processor, being configured to perform a method for processing scanning data which is applied to the above-described scanning data processing device, the method including:

acquiring local multi-frame three-dimensional data of an oral cavity and standard three-dimensional data of an auxiliary member; and
performing optimized splicing on the local multi-frame three-dimensional data of the oral cavity and the standard three-dimensional data of the auxiliary member to obtain overall three-dimensional data of the oral cavity.

Certainly, a storage medium provided by an embodiment of the present disclosure includes computer-executable instructions, and the computer-executable instructions thereof are not limited to the operation of the method as described above, and can also perform the relevant operation in the method for processing scanning data provided in any embodiment of the present disclosure.

According to the above descriptions about the implementation, a person skilled in the art may clearly learn that the present disclosure may be implemented by relying on software and essential common hardware, certainly, by using hardware, however, in many cases the former is the better way to implement the method. Based on such an understanding, the technical solutions of the present disclosure essentially, or the part contributing to the prior art, may be presented in the form of a software product. The computer software product may be stored in a computer-readable storage medium, such as a computer floppy disk, a Read-Only Memory (ROM), a Random Access Memory (RAM), a Flash, a hard disk or an optical disk, etc., and includes a number of instructions for causing a computer device (which may be a personal computer, a server, or a network device, etc.) to perform the method for processing scanning data provided in various embodiments of the present disclosure.

Note that the foregoing is only a preferred embodiment of the present disclosure and the technical principles utilized. It will be understood by those skilled in the art that the present disclosure is not limited to the particular embodiments described herein, and that various obvious variations, readjustments, and substitutions can be made by those skilled in the art without departing from the scope of protection of the present disclosure. Therefore, although the present disclosure is described in detail by the above embodiments, the present disclosure is not limited to the above embodiments, but may include other embodiments without departing from the concept of the present disclosure, and the scope of the present invention is determined by the scope of the appended claims.

**Industrial Applicability**

The three-dimensional scanning system provided by the present disclosure can achieve the purposes of reducing the optimization cost, and improving the accuracy of the full dental arch, and meets the accuracy requirements of multiple-missing-position or edentulous jaw implant bridges, thereby facilitating popularization and application, and being high industrially applicable.

**Claims**

1. A three-dimensional scanning system, comprising:

a three-dimensional scanning device (10) and auxiliary members (20);

the auxiliary member (20) is configured to be mounted on an implant of a dental arch in an oral cavity; and

the three-dimensional scanning device (10) is configured to scan the oral cavity to obtain local multi-frame three-dimensional data of the oral cavity, and to determine, on the basis of the local multi-frame three-dimensional data of the oral cavity and standard three-dimensional data of the auxiliary member (20), overall three-dimensional data of the oral cavity;

**characterized in that** the three-dimensional scanning device (10) comprises: an intraoral scanner (101) and a scanning data processing device (102);

the scanning data processing device (102) is configured to determine, on the basis of the local multi-frame three-dimensional data of the oral cavity and the standard three-dimensional data of the auxiliary member (20), the overall three-dimensional data of the oral cavity, wherein the standard three-dimensional data of the auxiliary member (20) refers to feature point data of each polygon on the auxiliary member (20) determined by the scanning data processing device (102) when designing the auxiliary member (20);

wherein the scanning data processing device (102) is configured to determine, on the basis of the local multi-frame three-dimensional data of the oral cavity and the standard three-dimensional data of the auxiliary member (20), the overall three-dimensional data of the oral cavity by:

> performing, by the scanning data processing device (102), optimized splicing on the local multi-frame three-dimensional data of the oral cavity and the standard three-dimensional data of the auxiliary member (20) to obtain the overall three-dimensional data of the oral cavity;
>
> wherein the scanning data processing device (102) is configured to perform optimized splicing on the local multi-frame three-dimensional data of the oral cavity and the standard three-dimensional data of the auxiliary member (20) to obtain the overall three-dimensional data of the oral cavity by:
>
>> performing sampled splicing on the local multi-frame three-dimensional data of the oral cavity and the standard three-dimensional data of the auxiliary member (20) to obtain a relative motion value of current sampled splicing and an average value of a distance between registration point pairs in the three-dimensional data of current sampled splicing;

constructing, on the basis of the relative motion value of current sampled splicing, the average value of a distance between registration point pairs in the three-dimensional data of current sampled splicing, and a relative motion value obtained from previous splicing, a global optimization energy function;

in the case where the global optimization energy function satisfies an iteration stopping condition and the relative motion value obtained from current splicing satisfies a viewing angle constraint condition, using the relative motion value obtained from current splicing as a relative motion target value; and

splicing, on the basis of the relative motion target value, the local multi-frame three-dimensional data of the oral cavity to determine the overall three-dimensional data of the oral cavity.

2. The three-dimensional scanning system as claimed in claim 1, wherein

the intraoral scanner (101) is configured to scan the oral cavity to obtain local multi-frame two-dimensional data of the oral cavity;

the scanning data processing device (102) is configured to perform three-dimensional reconstruction on the local multi-frame two-dimensional data of the oral cavity to obtain the local multi-frame three-dimensional data of the oral cavity.

3. The three-dimensional scanning system as claimed in claim 1, the auxiliary member (20) is configured to be mounted on an implant of a dental arch in an oral cavity, and the auxiliary member is provided with a mounting portion adapted to the implant; and

the auxiliary member (20) is a polyhedron composed of a plurality of irregular polygons, each polygon on the same auxiliary member has a different side length value.

4. The three-dimensional scanning system as claimed in claim 3, wherein a surface of the auxiliary member (20) has no reflective layer.

5. The three-dimensional scanning system as claimed in claim 1, wherein the scanning data processing device (102) is configured to perform, optimized splicing on the local multi-frame three-dimensional data of the oral cavity and the standard three-dimensional data of the auxiliary member to obtain the overall three-dimensional data of the oral cavity by:

performing, by the scanning data processing device (102), registration on the local multi-frame three-dimensional data of the oral cavity, as well as performing registration on the local multi-frame three-dimensional data of the oral cavity and the standard three-dimensional data of the auxiliary member, to obtain registration point pairs composed of the local multi-frame three-dimensional data and registration point pairs composed of the standard three-dimensional data of the auxiliary member and the local multi-frame three-dimensional data;

splicing the registration point pairs of the local multi-frame three-dimensional data of the oral cavity and the registration point pairs of the standard three-dimensional data of the auxiliary member to obtain spliced local multi-frame three-dimensional data of a whole oral cavity, and the spliced local multi-frame three-dimensional data comprises: three-dimensional data of the dental arch and the standard three-dimensional data of the auxiliary member. Optimizing, on the basis of the standard three-dimensional data of the auxiliary member;

optimizing, on the basic of standard three-dimensional data of the auxiliary member, the spliced local multi-frame three-dimensional data, to determine the overall three-dimensional data of the oral cavity.

6. The three-dimensional scanning system as claimed in claim 1, wherein the intraoral scanner (101) comprises a projection apparatus (11), an image acquisition apparatus (12) and an optical path adjustment apparatus (13), the optical path adjustment apparatus (13) is configured to change a transmission path of the structured light pattern to project the structured light pattern to the dental arch and project the structured light pattern modulated by the dental arch to the image acquisition apparatus (12), the image acquisition apparatus (12) is configured to perform beam splitting on the structured light pattern modulated by the dental arch, and acquire, by different cameras, a plurality of structured light patterns after beam splitting to obtain two-dimensional scanning data of the dental arch.

7. The three-dimensional scanning system as claimed in claim 6, wherein the projection apparatus (11) comprises an emitting apparatus (110), a light collimating apparatus (120) and a pattern forming apparatus (130), the emitting apparatus (110) is configured to emit preset light rays, and the preset light rays comprise at least two monochromatic light rays, the light collimating apparatus (120) is configured to homogenize the preset light rays; the pattern forming apparatus (130) is configured to project homogenized preset light rays in a structured light pattern.

8. The three-dimensional scanning system as claimed in claim 7, wherein the light collimating apparatus (120) comprises at least one fly-eye lens, and the fly-eye lens homogenizes the preset light rays to project the homogenized preset light rays to the pattern forming apparatus (130).

9. A method for processing scanning data, being applied to the three-dimensional scanning device in the three-dimensional scanning system as claimed in claims 1-8, the method comprising:

acquiring local multi-frame three-dimensional data of an oral cavity and standard three-dimensional data of an auxiliary member (20), wherein the standard three-dimensional data of the auxiliary member (20) refers to feature point data of each polygon on the auxiliary member (20) determined by the scanning data processing device when designing the auxiliary member (20); and

performing optimized splicing on the local multi-frame three-dimensional data of the oral cavity and the standard three-dimensional data of the auxiliary member (20) to obtain overall three-dimensional data of the oral cavity;

characterized in that the step of performing optimized splicing on the local multi-frame three-dimensional data of the oral cavity and the standard three-dimensional data of the auxiliary member (20) to obtain overall three-dimensional data of the oral cavity comprises:

performing sampled splicing on the local multi-frame three-dimensional data of the oral cavity and the standard three-dimensional data of the auxiliary member (20) to obtain a relative motion value of current sampled splicing and an average value of a distance between registration point pairs in the three-dimensional data of current sampled splicing;

constructing, on the basis of the relative motion value of current sampled splicing, the average value of a distance between registration point pairs in the three-dimensional data of current sampled splicing, and a relative motion value obtained from previous splicing, a global optimization energy function;

in the case where the global optimization energy function satisfies an iteration stopping condition and the relative motion value obtained from current splicing satisfies a viewing angle constraint condition, using the relative motion value obtained from current splicing as a relative motion target value; and

splicing, on the basis of the relative motion target value, the local multi-frame three-dimensional data of the oral cavity to determine the overall three-dimensional data of the oral cavity.

10. The method as claimed in claim 9, wherein the relative motion value obtained from previous splicing comprises: a relative motion initial value; and before constructing, on the basis of the relative motion value of current sampled splicing, the average value of the distance between registration point pairs in the three-dimensional data of current sampled splicing, and the relative motion value obtained from previous splicing, the global optimization energy function, the method further comprises:

fully splicing the local multi-frame three-dimensional data of the oral cavity and the standard three-dimensional data of the auxiliary member to determine the relative motion initial value.

11. The method as claimed in claim 9, wherein the iteration stopping condition comprises a preset threshold value, and the viewing angle constraint condition comprises a preset relative motion relationship; and

before using the relative motion value obtained from current splicing as the relative motion target value, the method further comprises:

determining whether a value of the global optimization energy function is less than or equal to the preset threshold value;

determining whether the relative motion value satisfies the preset relative motion relationship; and

in the case where the value of the global optimization energy function is less than or equal to the preset threshold value and the relative motion value satisfies the preset relative motion relationship, determining that the global optimization energy function satisfies the iteration stopping condition and the relative motion value obtained from current splicing satisfies the viewing angle constraint condition.

**Patentansprüche**

1. Dreidimensionales Scansystem, umfassend:

eine dreidimensionale Scanvorrichtung (10) und Hilfselemente (20);
wobei das Hilfselement (20) konfiguriert ist, um an einem Implantat eines Zahnbogens in einer Mundhöhle angebracht zu werden; und
die dreidimensionale Scanvorrichtung (10) konfiguriert ist, um die Mundhöhle zu scannen, um lokale dreidimensionale Mehrfachrahmendaten der Mundhöhle zu erhalten, und um auf der Basis der lokalen dreidimensionalen Mehrfachrahmendaten der Mundhöhle und von dreidimensionalen Standarddaten des Hilfselements (20) dreidimensionale Gesamtdaten der Mundhöhle zu bestimmen;
**dadurch gekennzeichnet, dass** die dreidimensionale Scanvorrichtung (10) umfasst:

einen Intraoralscanner (101) und
eine Scandatenverarbeitungsvorrichtung (102);
wobei die Scandatenverarbeitungsvorrichtung (102) konfiguriert ist, um auf der Basis der lokalen dreidimensionalen Mehrfachrahmendaten der Mundhöhle und der dreidimensionalen Standarddaten des Hilfselements (20) die dreidimensionalen Gesamtdaten der Mundhöhle zu bestimmen, wobei sich die dreidimensionalen Standarddaten des Hilfselements (20) auf Merkmalspunktdaten jedes Polygons auf dem Hilfselement (20) beziehen, die durch die Scandatenverarbeitungsvorrichtung (102) bestimmt werden, wenn das Hilfselement (20) entworfen wird;
wobei die Scandatenverarbeitungsvorrichtung (102) konfiguriert ist, um auf der Basis der lokalen dreidimensionalen Mehrfachrahmendaten der Mundhöhle und der dreidimensionalen Standarddaten des Hilfselements (20) die dreidimensionalen Gesamtdaten der Mundhöhle zu bestimmen durch:

Durchführen, durch die Scandatenverarbeitungsvorrichtung (102), eines optimierten Spleißens an den lokalen dreidimensionalen Mehrfachrahmendaten der Mundhöhle und den dreidimensionalen Standarddaten des Hilfselements (20), um die dreidimensionalen Gesamtdaten der Mundhöhle zu erhalten;
wobei die Scandatenverarbeitungsvorrichtung (102) konfiguriert ist, um das optimierte Spleißen an den lokalen dreidimensionalen Mehrfachrahmendaten der Mundhöhle und den dreidimensionalen Standarddaten des Hilfselements (20) durchzuführen, um die dreidimensionalen Gesamtdaten der Mundhöhle zu erhalten durch:

Durchführen eines abgetasteten Spleißens an den lokalen dreidimensionalen Mehrfachrahmendaten der Mundhöhle und den dreidimensionalen Standarddaten des Hilfselements (20), um einen Relativbewegungswert des aktuellen abgetasteten Spleißens und einen Mittelwert eines Abstands zwischen Registrierungspunktpaaren in den dreidimensionalen Daten des aktuellen abgetasteten Spleißens zu erhalten;

Konstruieren, auf der Basis des Relativbewegungswerts des aktuellen abgetasteten Spleißens, des Mittelwerts eines Abstands zwischen Registrierungspunktpaaren in den dreidimensionalen Daten des aktuellen abgetasteten Spleißens und eines Relativbewegungswerts, der aus dem vorherigen Spleißen erhalten wird, einer globalen Optimierungsenergiefunktion;

in dem Fall, in dem die globale Optimierungsenergiefunktion eine Iterationsstoppbedingung erfüllt und der Relativbewegungswert, der aus dem aktuellen Spleißen erhalten wird, eine Betrachtungswinkeleinschränkungsbedingung erfüllt, Verwenden des Relativbewegungswerts, der aus dem aktuellen Spleißen erhalten wird, als einen Relativbewegungszielwert; und

Spleißen, auf der Basis des Relativbewegungszielwerts, der lokalen dreidimensionalen Mehrfachrahmendaten der Mundhöhle, um die dreidimensionalen Gesamtdaten der Mundhöhle zu bestimmen.

2. Dreidimensionales Scansystem nach Anspruch 1, wobei

der Intraoralscanner (101) konfiguriert ist, um die Mundhöhle zu scannen, um lokale zweidimensionale Mehrfachrahmendaten der Mundhöhle zu erhalten;

die Scandatenverarbeitungsvorrichtung (102) konfiguriert ist, um eine dreidimensionale Rekonstruktion an den lokalen zweidimensionalen Mehrfachrahmendaten der Mundhöhle durchzuführen, um die lokalen dreidimensionalen Mehrfachrahmendaten der Mundhöhle zu erhalten.

3. Dreidimensionales Scansystem nach Anspruch 1, wobei das Hilfselement (20) konfiguriert ist, um an einem Implantat eines Zahnbogens in einer Mundhöhle angebracht zu werden, und das Hilfselement mit einem Anbringungsabschnitt bereitgestellt wird, der an das Implantat angepasst ist; und

das Hilfselement (20) ein Polyeder ist, das aus einer Vielzahl von unregelmäßigen Polygonen zusammengesetzt ist, wobei jedes Polygon auf demselben Hilfselement einen unterschiedlichen Seitenlängenwert aufweist.

4. Dreidimensionales Scansystem nach Anspruch 3, wobei eine Oberfläche des Hilfselements (20) keine reflektierende Schicht aufweist.

5. Dreidimensionales Scansystem nach Anspruch 1, wobei die Scandatenverarbeitungsvorrichtung (102) konfiguriert ist, um das optimierte Spleißen an den lokalen dreidimensionalen Mehrfachrahmendaten der Mundhöhle und den dreidimensionalen Standarddaten des Hilfselements durchzuführen, um die dreidimensionalen Gesamtdaten der Mundhöhle zu erhalten durch:

Durchführen, durch die Scandatenverarbeitungsvorrichtung (102), einer Registrierung an den lokalen dreidimensionalen Mehrfachrahmendaten der Mundhöhle, sowie Durchführen der Registrierung an den lokalen dreidimensionalen Mehrfachrahmendaten der Mundhöhle und den dreidimensionalen Standarddaten des Hilfselements, um Registrierungspunktpaare, die aus den lokalen dreidimensionalen Mehrfachrahmendaten zusammengesetzt sind, und Registrierungspunktpaare zu erhalten, die aus den dreidimensionalen Standarddaten des Hilfselements und den lokalen dreidimensionalen Mehrfachrahmendaten zusammengesetzt sind;

Spleißen der Registrierungspunktpaare der lokalen dreidimensionalen Mehrfachrahmendaten der Mundhöhle und der Registrierungspunktpaare der dreidimensionalen Standarddaten des Hilfselements, um gespleißte lokale dreidimensionale Mehrfachrahmendaten einer gesamten Mundhöhle zu erhalten, und die gespleißten lokalen dreidimensionalen Mehrfachrahmendaten umfassen: dreidimensionale Daten des Zahnbogens und die dreidimensionalen Standarddaten des Hilfselements. Optimieren, auf der Basis der dreidimensionalen Standarddaten des Hilfselements;

Optimieren, auf der Basis von dreidimensionalen Standarddaten des Hilfselements, der zusammengefügten lokalen dreidimensionalen Mehrfachrahmendaten, um die dreidimensionalen Gesamtdaten der Mundhöhle zu bestimmen.

6. Dreidimensionales Scansystem nach Anspruch 1, wobei der Intraoralscanner (101) eine Projektionseinrichtung (11), eine Bilderfassungseinrichtung (12) und eine Strahlenganganpassungseinrichtung (13) umfasst, die Strahlenganganpassungseinrichtung (13) konfiguriert ist, um einen Übertragungsweg des strukturierten Lichtmusters zu ändern, um das strukturierte Lichtmuster auf den Zahnbogen zu projizieren und das strukturierte Lichtmuster, das durch den Zahnbogen moduliert wird, auf die Bilderfassungseinrichtung (12) zu projizieren, die Bilderfassungseinrichtung (12) konfiguriert ist, um eine Strahlteilung an dem strukturierten Lichtmuster durchzuführen, das durch den Zahnbogen moduliert wird, und durch verschiedene Kameras eine Vielzahl von strukturierten Lichtmustern nach der Strahlteilung zu erfassen, um zweidimensionale Scandaten des Zahnbogens zu erhalten.

7. Dreidimensionales Scansystem nach Anspruch 6, wobei die Projektionseinrichtung (11) eine Abgabeeinrichtung (110), eine Lichtkollimationseinrichtung (120) und eine Musterbildungseinrichtung (130) umfasst, die Abgabeeinrichtung (110) konfiguriert ist, um voreingestellte Lichtstrahlen abzugeben, und die voreingestellten Lichtstrahlen mindestens zwei monochromatische Lichtstrahlen umfassen, die Lichtkollimationseinrichtung (120) konfiguriert ist, um die voreingestellten Lichtstrahlen zu homogenisieren; die Musterbildungseinrichtung (130) konfiguriert ist, um homogenisierte voreingestellte Lichtstrahlen in einem strukturierten Lichtmuster zu projizieren.

8. Dreidimensionales Scansystem nach Anspruch 7, wobei die Lichtkollimationseinrichtung (120) mindestens eine Fliegenaugenlinse umfasst, und die Fliegenaugenlinse die voreingestellten Lichtstrahlen homogenisiert, um die homogenisierten voreingestellten Lichtstrahlen auf die Musterbildungseinrichtung (130) zu projizieren.

9. Verfahren zum Verarbeiten von Scandaten, das auf die dreidimensionale Scanvorrichtung in dem dreidimensionalen Scansystem nach den Ansprüchen 1 bis 8 angewendet wird, das Verfahren umfassend:

    Erfassen lokaler dreidimensionaler Mehrfachrahmendaten einer Mundhöhle und dreidimensionaler Standarddaten eines Hilfselements (20), wobei sich die dreidimensionalen Standarddaten des Hilfselements (20) auf Merkmalspunktdaten jedes Polygons auf dem Hilfselement (20) beziehen, die durch die Scandatenverarbeitungsvorrichtung bestimmt werden, wenn das Hilfselement (20) entworfen wird; und

Durchführen des optimierten Spleißens an den lokalen dreidimensionalen Mehrfachrahmendaten der Mundhöhle und den dreidimensionalen Standarddaten des Hilfselements (20), um dreidimensionale Gesamtdaten der Mundhöhle zu erhalten;

**dadurch gekennzeichnet, dass der** Schritt des Durchführens des optimierten Spleißens an den lokalen dreidimensionalen Mehrfachrahmendaten der Mundhöhle und den dreidimensionalen Standarddaten des Hilfselements (20), um dreidimensionale Gesamtdaten der Mundhöhle zu erhalten, umfasst:

    Durchführen eines abgetasteten Spleißens an den lokalen dreidimensionalen Mehrfachrahmendaten der Mundhöhle und den dreidimensionalen Standarddaten des Hilfselements (20), um einen Relativbewegungswert des aktuellen abgetasteten Spleißens und einen Mittelwert eines Abstands zwischen Registrierungspunktpaaren in den dreidimensionalen Daten des aktuellen abgetasteten Spleißens zu erhalten;

Konstruieren, auf der Basis des Relativbewegungswerts des aktuellen abgetasteten Spleißens, des Mittelwerts eines Abstands zwischen Registrierungspunktpaaren in den dreidimensionalen Daten des aktuellen abgetasteten Spleißens und eines Relativbewegungswerts, der aus dem vorherigen Spleißen erhalten wird, einer globalen Optimierungsenergiefunktion;

in dem Fall, in dem die globale Optimierungsenergiefunktion eine Iterationsstoppbedingung erfüllt und der Relativbewegungswert, der aus dem aktuellen Spleißen erhalten wird, eine Betrachtungswinkeleinschränkungsbedingung erfüllt, Verwenden des Relativbewegungswerts, der aus dem aktuellen Spleißen erhalten wird, als einen Relativbewegungszielwert; und

Spleißen, auf der Basis des Relativbewegungszielwerts, der lokalen dreidimensionalen Mehrfachrahmendaten der Mundhöhle, um die dreidimensionalen Gesamtdaten der Mundhöhle zu bestimmen.

10. Verfahren nach Anspruch 9, wobei der Relativbewegungswert, der aus dem vorherigen Spleißen erhalten wird, umfasst: einen Relativbewegungsanfangswert; und

vor dem Konstruieren, auf der Basis des Relativbewegungswerts des aktuellen abgetasteten Spleißens, des Mittelwerts des Abstands zwischen Registrierungspunktpaaren in den dreidimensionalen Daten des aktuellen abgetasteten Spleißens und des Relativbewegungswerts, der aus dem vorherigen Spleißen erhalten wird, der globalen Optimierungsenergiefunktion, das Verfahren ferner umfasst:

vollständiges Spleißen der lokalen dreidimensionalen Mehrfachrahmendaten der Mundhöhle und der dreidimensionalen Standarddaten des Hilfselements, um den Relativbewegungsanfangswert zu bestimmen.

11. Verfahren nach Anspruch 9, wobei die Iterationsstoppbedingung einen voreingestellten Grenzwert umfasst und die Betrachtungswinkeleinschränkungsbedingung eine voreingestellte Relativbewegungsbeziehung umfasst; und
bevor der Relativbewegungswert, der aus dem aktuellen Spleißen erhalten wird, als der Relativbewegungszielwert verwendet wird, das Verfahren ferner umfasst:

Bestimmen, ob ein Wert der globalen Optimierungsenergiefunktion kleiner als oder gleich dem voreingestellten Grenzwert ist;
Bestimmen, ob der Relativbewegungswert die voreingestellte Relativbewegungsbeziehung erfüllt; und
in dem Fall, in dem der Wert der globalen Optimierungsenergiefunktion kleiner als oder gleich dem voreingestellten Grenzwert ist und der Relativbewegungswert die voreingestellte Relativbewegungsbeziehung erfüllt, Bestimmen, dass die globale Optimierungsenergiefunktion die Iterationsstoppbedingung erfüllt und der Relativbewegungswert, der aus dem aktuellen Spleißen erhalten wird, die Betrachtungswinkeleinschränkungsbedingung erfüllt.

## Revendications

1. Système de balayage tridimensionnel, comprenant :

un dispositif de balayage tridimensionnel (10) et des éléments auxiliaires (20) ;
l'élément auxiliaire (20) est configuré pour être monté sur un implant d'une arcade dentaire dans une cavité buccale ; et
le dispositif de balayage tridimensionnel (10) est configuré pour balayer la cavité buccale pour obtenir des données tridimensionnelles multitrames locales de la cavité buccale et pour déterminer, sur la base des données tridimensionnelles multi-trames locales de la cavité buccale et de données tridimensionnelles standard de l'élément auxiliaire (20), des données tridimensionnelles d'ensemble de la cavité buccale ; **caractérisé en ce que** le dispositif de balayage tridimensionnel (10) comprend :

un scanner intrabuccal (101) et
un dispositif de traitement de données de balayage (102) ;
le dispositif de traitement de données de balayage (102) est configuré pour déterminer, sur la base des données tridimensionnelles multi-trames locales de la cavité buccale et des données tridimensionnelles standard de l'élément auxiliaire (20), les données tridimensionnelles d'ensemble de la cavité buccale, dans lequel les données tridimensionnelles standard de l'élément auxiliaire (20) font référence à des données de points caractéristiques de chaque polygone sur l'élément auxiliaire (20) déterminées par le dispositif de traitement de données de balayage (102) lors de la conception de l'élément auxiliaire (20) ;
dans lequel le dispositif de traitement de données de balayage (102) est configuré pour déterminer, sur la base des données tridimensionnelles multi-trames locales de la cavité buccale et des données tridimensionnelles standard de l'élément auxiliaire (20), les données tridimensionnelles d'ensemble de la cavité buccale par :

la réalisation, par le dispositif de traitement de données de balayage (102), d'un collage optimisé sur les données tridimensionnelles multi-trames locales de la cavité buccale et les données tridimensionnelles standard de l'élément auxiliaire (20) pour obtenir les données tridimensionnelles d'ensemble de la cavité buccale ;
dans lequel le dispositif de traitement de données de balayage (102) est configuré pour réaliser un collage optimisé sur les données tridimensionnelles multi-trames locales de la cavité buccale et les données tridimensionnelles standard de l'élément auxiliaire (20) pour obtenir les données tridimensionnelles d'ensemble de la cavité buccale par :

la réalisation d'un collage échantillonné sur les données tridimensionnelles multi-trames locales de la cavité buccale et les données tridimensionnelles standard de l'élément auxiliaire (20) pour obtenir une valeur de mouvement relatif du collage échantillonné actuel et une valeur moyenne d'une distance entre des paires de points d'enregistrement dans les données tridimensionnelles du collage échantillonné actuel ;

la construction, sur la base de la valeur de mouvement relatif du collage échantillonné actuel, de la valeur moyenne d'une distance entre des paires de points d'enregistrement dans les données tridimensionnelles du collage échantillonné actuel ; et d'une valeur de mouvement relatif obtenue à partir du collage précédent, d'une fonction d'énergie d'optimisation globale ;

dans le cas où la fonction d'énergie d'optimisation globale satisfait à une condition d'arrêt d'itération et où la valeur de mouvement relatif obtenue à partir du collage actuel satisfait à une condition de contrainte d'angle de vue, l'utilisation de la valeur de mouvement relatif obtenue à partir du collage actuel comme valeur cible de mouvement relatif ; et

le collage, sur la base de la valeur cible de mouvement relatif, des données tridimensionnelles multi-trames locales de la cavité buccale pour déterminer les données tridimensionnelles d'ensemble de la cavité buccale.

2. Système de balayage tridimensionnel selon la revendication 1, dans lequel

le scanner intrabuccal (101) est configuré pour balayer la cavité buccale pour obtenir des données bidimensionnelles multi-trames locales de la cavité buccale ;

le dispositif de traitement de données de balayage (102) est configuré pour réaliser une reconstruction tridimensionnelle sur les données bidimensionnelles multi-trames locales de la cavité buccale pour obtenir les données tridimensionnelles multi-trames locales de la cavité buccale.

3. Système de balayage tridimensionnel selon la revendication 1, l'élément auxiliaire (20) est configuré pour être monté sur un implant d'une arcade dentaire dans une cavité buccale, et l'élément auxiliaire est pourvu d'une partie de montage adaptée à l'implant ; et

l'élément auxiliaire (20) est un polyèdre composé d'une pluralité de polygones irréguliers, chaque polygone sur le même élément auxiliaire a une valeur de longueur de côté différente.

4. Système de balayage tridimensionnel selon la revendication 3, dans lequel une surface de l'élément auxiliaire (20) ne possède pas de couche réfléchissante.

5. Système de balayage tridimensionnel selon la revendication 1, dans lequel le dispositif de traitement de données de balayage (102) est configuré pour réaliser un collage optimisé sur les données tridimensionnelles multi-trames locales de la cavité buccale et les données tridimensionnelles standard de l'élément auxiliaire pour obtenir les données tridimensionnelles d'ensemble de la cavité buccale par :

la réalisation, par le dispositif de traitement de données de balayage (102), d'un enregistrement sur les données tridimensionnelles multi-trames locales de la cavité buccale, ainsi que la réalisation d'un enregistrement sur les données tridimensionnelles multi-trames locales de la cavité buccale et les données tridimensionnelles standard de l'élément auxiliaire, pour obtenir des paires de points d'enregistrement composées des données tridimensionnelles multi-trames locales et des paires de points d'enregistrement composées des données tridimensionnelles standard de l'élément auxiliaire et des données tridimensionnelles multi-trames locales ;

le collage des paires de points d'enregistrement des données tridimensionnelles multi-trames locales de la cavité buccale et des paires de points d'enregistrement des données tridimensionnelles standard de l'élément auxiliaire pour obtenir des données tridimensionnelles multi-trames locales collées d'une cavité buccale entière, et les données tridimensionnelles multi-trames locales collées comprennent : des données tridimensionnelles de l'arcade dentaire et les données tridimensionnelles standard de l'élément auxiliaire. L'optimisation, sur la base des données tridimensionnelles standard de l'élément auxiliaire ;

l'optimisation, sur la base des données tridimensionnelles standard de l'élément auxiliaire, des données tridimensionnelles multi-trames locales collées, pour déterminer les données tridimensionnelles d'ensemble de la cavité buccale.

6. Système de balayage tridimensionnel selon la revendication 1, dans lequel le scanner intrabuccal (101) comprend un appareil de projection (11), un appareil d'acquisition d'images (12) et un appareil de réglage du trajet optique (13), l'appareil de réglage du trajet optique (13) est configuré pour modifier un trajet de transmission du motif lumineux structuré pour projeter le motif lumineux structuré sur l'arcade dentaire et projeter le motif lumineux structuré modulé par l'arcade dentaire sur l'appareil d'acquisition d'images (12), l'appareil d'acquisition d'images (12) est configuré pour réaliser une division de faisceau sur le motif lumineux structuré modulé par l'arcade dentaire, et acquérir, par différentes caméras, une pluralité de motifs lumineux structurés après la division de faisceau pour obtenir des données de balayage bidimensionnelles de l'arcade dentaire.

7. Système de balayage tridimensionnel selon la revendication 6, dans lequel l'appareil de projection (11) comprend un appareil d'émission (110), un appareil de collimation de la lumière (120) et un appareil de formation de motif (130), l'appareil d'émission (110) est configuré pour émettre des rayons lumineux prédéfinis, et les rayons lumineux prédéfinis comprennent au moins deux rayons lumineux monochromatiques, l'appareil de collimation de la lumière (120) est configuré pour homogénéiser les rayons lumineux prédéfinis ; l'appareil de formation de motif (130) est configuré pour projeter des rayons lumineux homogénéisés prédéfinis dans un motif lumineux structuré.

8. Système de balayage tridimensionnel selon la revendication 7, dans lequel l'appareil de collimation de la lumière (120) comprend au moins une lentille en œil de mouche, et la lentille en œil de mouche homogénéise les rayons lumineux prédéfinis pour projeter les rayons lumineux prédéfinis homogénéisés vers l'appareil de formation de motif (130).

9. Procédé de traitement des données de balayage, appliqué au dispositif de balayage tridimensionnel dans le système de balayage tridimensionnel selon les revendications 1 à 8, le procédé comprenant :

   l'acquisition de données tridimensionnelles multi-trames locales d'une cavité buccale et de données tridimensionnelles standard d'un élément auxiliaire (20), dans lequel les données tridimensionnelles standard de l'élément auxiliaire (20) font référence à des données de points caractéristiques de chaque polygone sur l'élément auxiliaire (20) déterminées par le dispositif de traitement de données de balayage lors de la conception de l'élément auxiliaire (20) ; et
   la réalisation d'un collage optimisé sur les données tridimensionnelles multi-trames locales de la cavité buccale et les données tridimensionnelles standard de l'élément auxiliaire (20) pour obtenir des données tridimensionnelles d'ensemble de la cavité buccale ;
   **caractérisé en ce que** l'étape consistant à réaliser un collage optimisé sur les données tridimensionnelles multi-trames locales de la cavité buccale et les données tridimensionnelles standard de l'élément auxiliaire (20) pour obtenir des données tridimensionnelles d'ensemble de la cavité buccale comprend :

   la réalisation d'un collage échantillonné sur les données tridimensionnelles multi-trames locales de la cavité buccale et les données tridimensionnelles standard de l'élément auxiliaire (20) pour obtenir une valeur de mouvement relatif du collage échantillonné actuel et une valeur moyenne d'une distance entre des paires de points d'enregistrement dans les données tridimensionnelles du collage échantillonné actuel ;
   la construction, sur la base de la valeur de mouvement relatif du collage échantillonné actuel, de la valeur moyenne d'une distance entre des paires de points d'enregistrement dans les données tridimensionnelles du collage échantillonné actuel ; et d'une valeur de mouvement relatif obtenue à partir du collage précédent, d'une fonction d'énergie d'optimisation globale ;
   dans le cas où la fonction d'énergie d'optimisation globale satisfait à une condition d'arrêt d'itération et où la valeur de mouvement relatif obtenue à partir du collage actuel satisfait à une condition de contrainte d'angle de vue, l'utilisation de la valeur de mouvement relatif obtenue à partir du collage actuel comme valeur cible de mouvement relatif ; et
   le collage, sur la base de la valeur cible de mouvement relatif, des données tridimensionnelles multi-trames locales de la cavité buccale pour déterminer les données tridimensionnelles d'ensemble de la cavité buccale.

10. Procédé selon la revendication 9, dans lequel la valeur de mouvement relatif obtenue à partir du collage précédent comprend : une valeur initiale de mouvement relatif ; et
   avant de construire, sur la base de la valeur de mouvement relatif du collage échantillonné actuel, de la valeur moyenne de la distance entre des paires de points d'enregistrement dans les données tridimensionnelles du collage échantillonné actuel, et de la valeur de mouvement relatif obtenue à partir du collage précédent, la fonction d'énergie d'optimisation globale, le procédé comprend en outre :
   le collage complet des données tridimensionnelles multi-trames locales de la cavité buccale et des données tridimensionnelles standard de l'élément auxiliaire pour déterminer la valeur initiale du mouvement relatif.

11. Procédé selon la revendication 9, dans lequel la condition d'arrêt d'itération comprend une valeur seuil prédéfinie, et la condition de contrainte d'angle de vue comprend une relation de mouvement relatif prédéfinie ; et

avant d'utiliser la valeur de mouvement relatif obtenue à partir du collage actuel comme valeur cible de mouvement relatif, le procédé comprend en outre :

la détermination du fait de savoir si une valeur de la fonction d'énergie d'optimisation globale est inférieure ou égale à la valeur seuil prédéfinie ;

la détermination du fait de savoir si la valeur de mouvement relatif satisfait à la relation de mouvement relatif prédéfinie ; et

dans le cas où la valeur de la fonction d'énergie d'optimisation globale est inférieure ou égale à la valeur seuil prédéfinie et où la valeur de mouvement relatif satisfait à la relation de mouvement relatif prédéfinie, la détermination du fait que la fonction d'énergie d'optimisation globale satisfait à la condition d'arrêt d'itération et que la valeur de mouvement relatif obtenue à partir du collage actuel satisfait à la condition de contrainte d'angle de vue.

Missing range

20

Y: Height of a
dental arch

X: Width of a
dental arch

101

102

10

Intraoral scanner → Scanning data
processing device

Three-dimensional scanning device

FIG. 1

110

120

130

13

11

Emitting
apparatus → Light
collimating
apparatus → Pattern
forming
apparatus → Optical path
adjustment
apparatus

Projection apparatus

12

Image acquisition apparatus

FIG. 2

FIG. 3

| Acquire local multi-frame three-dimensional data of an oral cavity and standard three-dimensional data of an auxiliary member | ~ S410 |

| Perform optimized splicing on the local multi-frame three-dimensional data of the oral cavity and the standard three-dimensional data of the auxiliary member to obtain overall three-dimensional data of the oral cavity | ~ S420 |

FIG. 4

| Data acquisition component | ~ 510 |

| Overall three-dimensional data determination component | ~ 520 |

FIG. 5

600

Storage apparatus    610

Input apparatus    630

Output apparatus    640

Processor    620

FIG. 6

**REFERENCES CITED IN THE DESCRIPTION**

**Patent documents cited in the description**

- US 2021137653 A1
- US 2020405457 A1

- US 2021038350 A1